# FASCICULE DE BREVET EUROPEEN

(11) **EP 0 511 097 B1**
(45) Date de publication et mention de la délivrance du brevet: **24.01.1996**
(21) Numéro de dépôt: 92401144.8
(22) Date de dépôt: 22.04.1992
(51) Int. Cl.: G01N 1/10, G21F 7/06

(54) **Installation de prélèvement d'échantillons fluides dans une zone confinée**
Anordnung zur Entnahme von fliessenden Proben aus einer geschlossenen Zone
Installation for withdrawing fluid samples out of a confined zone

(30) Priorité: 23.04.1991 FR 9104995
(43) Date de publication de la demande: 28.10.1992
(73) Titulaire: COMPAGNIE GENERALE DES MATIERES NUCLEAIRES (COGEMA), F-78141 Vélizy-Villacoublay (FR)
(72) Inventeur: Conche, François, F-50100 Cherbourg (FR)
(74) Mandataire: Poulin, Gérard

(56) Documents cités:
- EP-A- 0 078 211
- EP-A- 0 079 283
- EP-A- 0 296 917
- FR-A- 2 515 350
- GB-A- 2 203 243

## Description

L'invention concerne une installation conçue pour effectuer à distance des prélèvements d'échantillons fluides tels que des liquides radioactifs à l'intérieur d'une zone confinée située en dessous d'une dalle de protection biologique.

Plusieurs installations de ce type peuvent notamment être implantées dans une usine de traitement de combustibles nucléaires irradiés, pour assurer de façon automatisée et à distance le prélèvement d'échantillons de solutions actives dans différentes parties de l'usine. Elles permettent d'introduire de manière contrôlée des échantillons fluides dans des flacons appelés cruchons, réalisés en matière plastique souple et préalablement mis sous vide, avant d'analyser ces échantillons en laboratoire.

Afin que la protection biologique du personnel contre les particules a et les rayons y soit assurée dans les meilleures conditions, sans avoir à donner au matériel utilisé une masse et un volume trop importants, il a été proposé dans le document FR-A-2 515 350 de réaliser les prélèvements directement à l'intérieur d'une cuve située sous la dalle de protection biologique. Pour cela, les cruchons traversent la dalle avant d'être piqués sur des aiguilles disposées dans le fond de la cuve et vers lesquelles sont acheminés différents fluides dont des prélèvements sont souhaités. A cet effet, chacune des aiguilles plonge, en dessous du fond de la cuve, dans un pot alimenté en fluide par un circuit tel que celui qui fait l'objet du document FR-A-2 516 242.

Dans une telle installation, la principale difficulté concerne le transfert des cruchons au travers de la dalle, ainsi que l'opération de prélèvement proprement dite, qui doit être réalisée en aveugle et de façon automatisée.

La solution à ces problèmes décrite dans le document FR-A-2 515 350 est une solution totalement mécanique. Ainsi, lorsqu'il a été introduit dans l'installation par un système de transfert pneumatique, le cruchon est poussé par un piston horizontal jusqu'à l'extrémité supérieure d'un puits dans lequel sa descente est commandée par la rotation d'une vis sans fin verticale traversant la dalle. Dans le bas du puits, le cruchon est repris par un barillet rotatif d'axe vertical, dont une rotation a pour effet d'amener le cruchon en dessous d'un outil de préhension traversant également la dalle. Cet outil de préhension commande la descente du cruchon et assure le prélèvement en venant piquer le cruchon sur l'aiguille. Pour pouvoir accéder à plusieurs aiguilles disposées selon un ou plusieurs arcs de cercle dans le fond de la cuve, cet ensemble est monté sur un bouchon tournant appartenant à la dalle et dont l'axe est confondu avec l'axe de ces arcs de cercle.

Un tel mécanisme est relativement complexe et présente des risques inacceptables de coincement des cruchons, notamment lorsque ces derniers sont poussés par le piston et lors de leur descente dans le puits contrôlé par la vis sans fin. On conçoit en effet que le coincement d'un cruchon dans la traversée de la dalle nécessiterait une intervention particulièrement délicate et dangereuse pour le personnel.

Par ailleurs, l'installation décrite dans ce document prévoit, lorsque le prélèvement a été effectué, une évacuation des cruchons par le fond de la cuve, difficilement compatible avec l'implantation habituelle des laboratoires d'analyses des échantillons prélevés en-dehors de la zone confinée. S'il est envisagé de pouvoir évacuer les cruchons remplis en les faisant à nouveau traverser la dalle à l'aide du mécanisme précédemment décrit, cela nécessiterait le recours à d'autres moyens non décrits, car le piston ne permet le transfert du cruchon que dans un sens.

L'invention a précisément pour objet une installation de prélèvement d'échantillons fluides utilisant également une cuve de prélèvement située en dessous de la dalle, mais dans laquelle la manutention à distance des cruchons permettant de traverser la dalle dans l'un et l'autre sens et d'effectuer les prélèvements est assurée d'une manière beaucoup plus simple et beaucoup plus sûre que dans l'art antérieur, de façon à réduire autant que possible les risques de coincement des cruchons et à permettre sans difficulté l'évacuation ues cruchons remplis vers un laboratoire d'analyses situé à l'extérieur de la zone confinée.

Conformément à l'invention, ce résultat est obtenu au moyen d'une installation de prélèvement d'échantillons fluides dans une zone confinée située sous une dalle de protection, cette installation comprenant une cuve de prélèvement située sous la dalle, au moins une aiguille de prélèvement traversant un fond de la cuve, et des moyens de manutention à distance pour transférer un à un des cruchons de prélèvement dans et hors de la cuve, au travers de la dalle, et pour piquer ces cruchons un à un sur ladite aiguille, afin d'effectuer un prélèvement, caractérisée par le fait que lesdits moyens de manutention à distance comprennent :
- un conduit de transfert des cruchons, traversant un élément de la dalle, mobile verticalement entre une position haute d'attente et une position basse de prélèvement ;
- un barillet monté rotatif sous ledit élément de dalle, autour d'un axe décalé par rapport audit conduit, et comportant une nacelle de réception de cruchon apte à être amenée, par rotation du barillet, dans une position angulaire de réception et d'évacuation de cruchon, dans laquelle la nacelle est placée sous le conduit, et dans au moins une position angulaire de prélèvement, dans laquelle la nacelle peut être amenée au-dessus de l'aiguille de prélèvement ; et
- des moyens de transfert pneumatique des cruchons, vers le bas et vers le haut, dans ledit conduit.

Dans une installation de prélèvement ainsi réalisée, les cruchons sous vide sont transférés directement dans la nacelle située sous la dalle par le système de transfert pneumatique. Une simple rotation du barillet amène la nacelle dans une position dans laquelle le prélèvement peut être directement effectué par une descente de l'élément de dalle portant le barillet, sans qu'il soit nécessaire d'avoir recours à un outil de préhension quelconque. Des mouvements inverses de l'élément de dalle et du barillet amènent à nouveau la nacelle dans laquelle se trouve le cruchon rempli à la verticale du conduit de transfert. Le cruchon rempli peut alors être acheminé par ce conduit jusqu'au laboratoire d'analyses sous l'action des moyens de transfert pneumatique.

Comme dans l'art antérieur, plusieurs aiguilles de prélèvement sont avantageusement disposées dans le fond de la cuve, selon au moins un arc de cercle d'axe décalé par rapport à l'axe du barillet. Un bouchon tournant, d'axe confondu avec l'axe de cet arc de cercle, forme alors au-dessus de la cuve une partie de la dalle et ledit élément de dalle est logé dans ce bouchon tournant, de telle sorte que, dans chaque position angulaire de prélèvement de la nacelle, cette dernière peut être amenée au-dessus de chacune des aiguilles par une rotation du bouchon tournant.

La rotation du barillet peut notamment être commandée par des moyens de commande montés sur ledit élément de dalle et agissant sur un arbre de commande traversant l'élément de dalle selon son axe et solidaire du barillet à son extrémité inférieure.

Les moyens de transfert pneumatique des cruchons peuvent comprendre une canalisation d'amenée d'air qui chemine dans ledit arbre de commande et débouche en dessous de la nacelle. Cette canalisation, qui communique habituellement avec des moyens de ventilation de la cuve au travers de premiers moyens formant vanne est reliée lorsqu'on désire évacuer un cruchon rempli, à des moyens d'amenée d'air comprimé au travers de deuxièmes moyens formant vanne.

Dans un mode de réalisation préféré de l'invention, chaque aiguille de prélèvement est emboîtée de façon démontable dans un réceptacle prévu dans le fond de la cuve. Des moyens de manutention à distance comprennent alors un organe anti-arrachement monté sur la nacelle de façon à pouvoir se déplacer sur celle-ci entre une position haute, dans laquelle cet organe affleure l'extrémité inférieure de la nacelle et une position basse dans laquelle cet organe est espacé de l'extrémité inférieure de la nacelle d'une distance sensiblement égale à la longueur d'une partie de chaque aiguille faisant saillie au-dessus du réceptacle. L'organe anti-arrachement reste ainsi en appui sur l'aiguille de prélèvement, par exemple sous l'action de moyens élastiques, lors de la remontée du cruchon consécutive à son piquage sur l'aiguille, tant que cette dernière reste enfoncée dans le cruchon, ce qui évite tout risque d'arrachement de l'aiguille à ce moment.

Dans un mode de réalisation préféré de l'invention, la canalisation d'amenée d'air comporte un tube rigide, logé de façon coulissante dans l'arbre de commande et portant l'organe anti-arrachement à son extrémité inférieure.

De préférence, afin de permettre le remplacement d'une aiguille usagée et la mise en place d'une aiguille neuve, l'élément de dalle comporte deux alvéoles dont l'une au moins peut être amenée au-dessus d'une aiguille et sous chacune desquelles peut être amenée la nacelle, par une rotation du barillet. Un outil de préhension est alors logé de façon coulissante dans chacune des alvéoles de façon à pouvoir saisir un bouchon de cruchon ou une aiguille.

Avantageusement, des moyens d'isolement sont placés au-dessus de la dalle et raccordés au conduit de transfert par un tube souple. Ces moyens d'isolement peuvent occuper une position d'isolement, dans laquelle le tube souple est isolé d'un tube de transfert placé au-dessus des moyens d'isolement, et une position de passage dans laquelle le tube souple et le tube de transfert communiquent entre eux.

Les moyens de transfert pneumatique des cruchons peuvent alors comprendre une deuxième canalisation d'amenée d'air, qui communique avec des moyens d'amenée d'air comprimé au travers de troisièmes moyens formant vanne et qui débouche dans le tube de transfert, immédiatement au-dessus des moyens d'isolement.

Pour permettre le fonctionnement automatique de l'installation, différents détecteurs sont prévus tels qu'un détecteur de présence d'un cruchon dans la nacelle et un détecteur de passage d'un cruchon dans la partie haute du conduit de transfert, au-dessus des moyens d'isolement.

On décrira maintenant , un mode de réalisation préféré de l'invention, en se référant aux dessins annexés, dans lesquels :
- La figure 1 est une vue de côté, en coupe partielle, représentant de façon schématique une installation de prélèvement d'échantillons fluides conformes à l'invention ;
- la figure 2 est une vue en perspective écorchée illustrant à plus grande échelle la partie de l'installation située au niveau de la dalle et au-dessous de celle-ci ;
- la figure 3 est une vue en coupe verticale représentant notamment le barillet rotatif portant la nacelle apte à recevoir le cruchon, ainsi que l'organe anti-arrachement associé à cette nacelle ;
- la figure 4 est une vue en perspective et en coupe verticale partielle illustrant schématiquement la position de l'élément de dalle servant à la traversée du cruchon, par rapport à l'agencement des aiguilles dans le fond de la cuve ;
- la figure 5 est une vue de dessus illustrant schématiquement les positions que peut occuper le conduit de transfert des cruchons par rapport aux aiguilles implantées dans le fond de la cuve ;
- la figure 6 est un schéma qui représente le circuit pneumatique de transfert des cruchons dans l'installation ;
- les figures 7A à 7F sont des vues en coupe verticale illustrant différentes phases de fonctionnement de l'installation de prélèvement selon l'invention, lorsque cette installation est mise en oeuvre pour réaliser un prélèvement ;
- les figures 8A et 8B sont des vues en coupe verticale illustrant la mise en place d'une aiguille à l'aide d'un outil de préhension incorporé dans l'installation de prélèvement selon l'invention ;
- les figures 9A à 9F sont des vues en coupe verticale illustrant schématiquement différentes phases de mise en oeuvre de l'installation de prélèvement selon l'invention, lors de l'enlèvement d'une aiguille usagée à l'aide de l'outil de préhension des figures 8A et 8B ; et
- les figures 10A à 10F sont des vues en coupe verticale illustrant schématiquement différentes phases de mise en oeuvre de l'installation de prélèvement selon l'invention, lors de la mise en place d'une aiguille neuve.

L'installation représentée sur les figures est conçue pour assurer le prélèvement automatique et à distance d'échantillons de solution active, dans une zone confinée 10 délimitée notamment vers le haut par une dalle horizontale 12 de protection biologique. A titre d'exemple, la zone confinée 10 peut abriter une unité chimique de traitement de combustibles nucléaires irradiés.

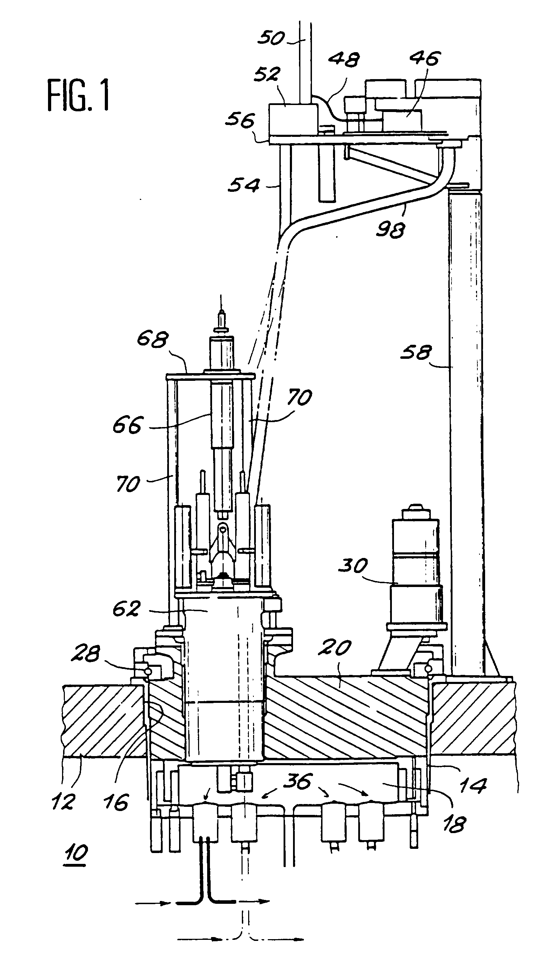

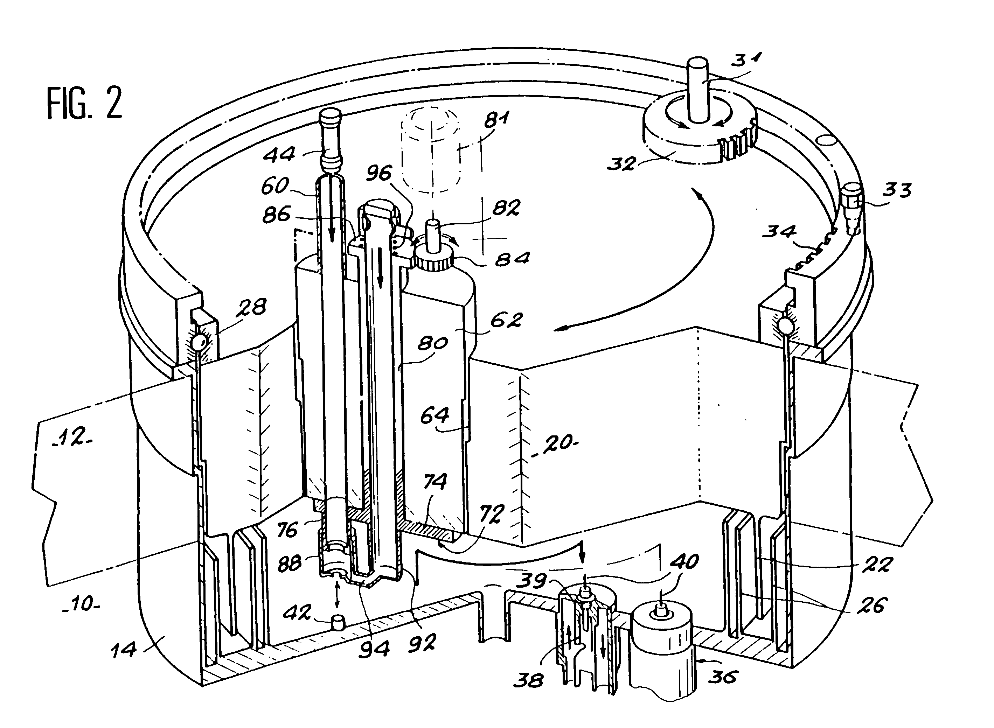

Comme le montrent notamment les figures 1 et 2, l'installation de prélèvement selon l'invention comprend une cuve cylindrique 14, d'axe vertical, placée dans une ouverture circulaire 16 formée dans la dalle 12 et suspendue à cette dernière, de façon à délimiter à l'intérieur de la zone confinée 10 un volume clos 18 fermé à sa partie supérieure par un bouchon tournant 20 formant une partie de la dalle de protection biologique 12.

L'étanchéité entre le bouchon tournant 20 et la cuve 14 est assurée par un joint hydraulique formé entre une paroi cylindrique 22, qui fait saillie vers le bas à la périphérie du bouchon 20 et une garde hydrauliquide dans laquelle plonge la paroi cylindrique 22 et qui est formée entre deux doubles parois cylindriques 24 qui font saillie vers le haut de part et d'autre de la paroi 22, à la périphérie du fond sensiblement plan et horizontal de la cuve 14.

Le bouchon tournant 20 est supporté de façon rotative dans la cuve 14, par exemple par un palier à billes 28. La commande de la rotation du bouchon 20 est assurée par un ensemble motoréducteur 30 (figure 1) monté sur la face supérieure du bouchon et dont l'arbre de sortie vertical 31 entraîne un pignon moteur 32 (figure 2) qui est en prise sur une couronne dentée 34 montée sur une bride supérieure de la cuve 14.

Afin d'assurer un positionnement angulaire précis du bouchon tournant 20, l'ensemble motoréducteur 30 comporte un variateur de vitesse à courant continu et une dynamo tachymétrique. De plus, le positionnement angulaire est connu grâce à un codeur, un verrou 33 étant actionné à chaque fois que le bouchon tournant atteint une position angulaire prédéterminée correspondant à la réalisation d'un prélèvement. Le bon fonctionnement de ce verrou, constitué d'un pène actionné par un moteur à courant continu, est contrôlé par un capteur de position et par un capteur à déplacement linéaire.

Comme l'illustrent notamment les figures 1 et 2, le fond de la cuve 14 est équipé de têtes de prélèvement 36 constituées essentiellement d'un pot de prélèvement 38 solidaire du fond de la cuve et d'une aiguille de prélèvement 40, dont le corps est emboîté de façon amovible dans un réceptacle 39 du pot de prélèvement, depuis l'intérieur de la cuve 14.

Chacun des pots de prélèvement 38 est relié, par un circuit qui ne fait pas partie de l'invention, à une partie déterminée de l'unité chimique qui se trouve à l'intérieur de la zone confinée 10, de telle sorte que des fluides différents, ou des fluides identiques prélevés à des endroits différents, circulent dans chacun des pots de prélèvement 38. L'emboîtement des aiguilles 40 dans les pots 38 est tel que la partie inférieure de chacune des aiguilles plonge dans le fluide correspondant.

Comme l'illustrent notamment les figures 2, 4 et 5, les têtes de prélèvement 36 sont disposées dans le fond de la cuve 14 selon deux arcs de cercle concentriques dont l'axe est confondu avec l'axe vertical de la cuve 14 et du bouchon tournant 20.

On trouve aussi dans le fond de la cuve 14 un plot de confinement 42, ayant la forme d'un pion cylindrique d'axe vertical, qui est disposé à égale distance des deux arcs de cercle sur lesquels sont disposées les têtes de prélèvement 36. Un deuxième plot de confinement 42, symétrique du premier par rapport à l'axe vertical de la cuve, est avantageusement prévu pour remplacer le premier, dans le cas où il serait endommagé.

L'installation de prélèvement d'échantillons selon l'invention est conçue pour prélever les échantillons fluides dans des cruchons 44, réalisés en matière plastique souple et préalablement mis sous vide, par piquage de ces cruchons sur l'une ou l'autre des aiguilles 40.

Dans la pratique, les cruchons 44 sont généralement constitués d'une enveloppe extérieure, appelée curseur, dont la forme est conçue pour permettre le transfert pneumatique des cruchons dans des tubes sans risque de coincement, et d'un cruchon proprement dit, placé à l'intérieur de cette enveloppe et comportant à son extrémité tournée vers le bas lorsqu'il pénètre dans l'installation un bouchon apte à être transpercé par les aiguilles 40 lors des prélèvements. Pour l'essentiel, cet agencement est comparable à celui qui est décrit dans le document FR-A-8 120 039, de sorte qu'il n'en sera pas fait de desciption détaillée.

Il est à noter qu'en variante, les cruchons 44 peuvent être réalisés d'un seul tenant, le cruchon proprement dit présentant alors la forme extérieure du curseur dans le cas précédent.

Les cruchons sous vide destinés à effectuer les prélèvements d'échantillons fluides sont expédiés un à un et à la demande vers l'installation de prélèvement selon l'invention depuis un poste de distribution (non représenté) qui ne fait pas partie de l'invention. Il est à noter que chacun des cruchons sous vide est avantageusement identifié par un marquage avant son expédition, afin que chaque cruchon 44 puisse être ensuite distingué des autres lors du prélèvement, puis des analyses effectuées sur les échantillons prélevés.

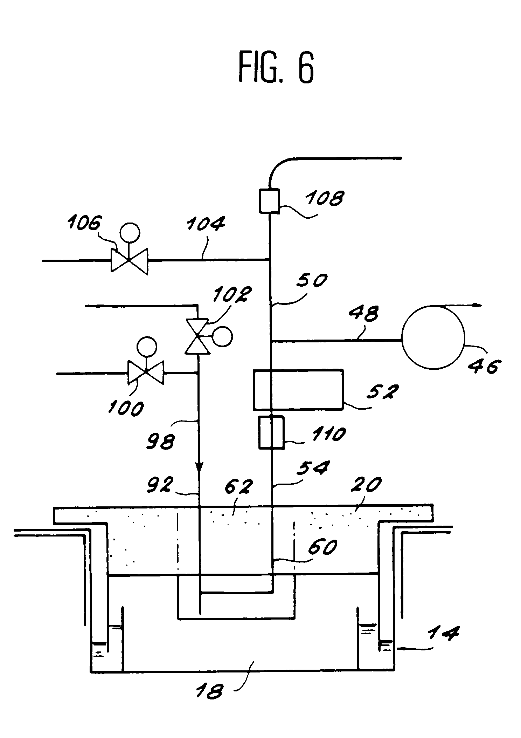

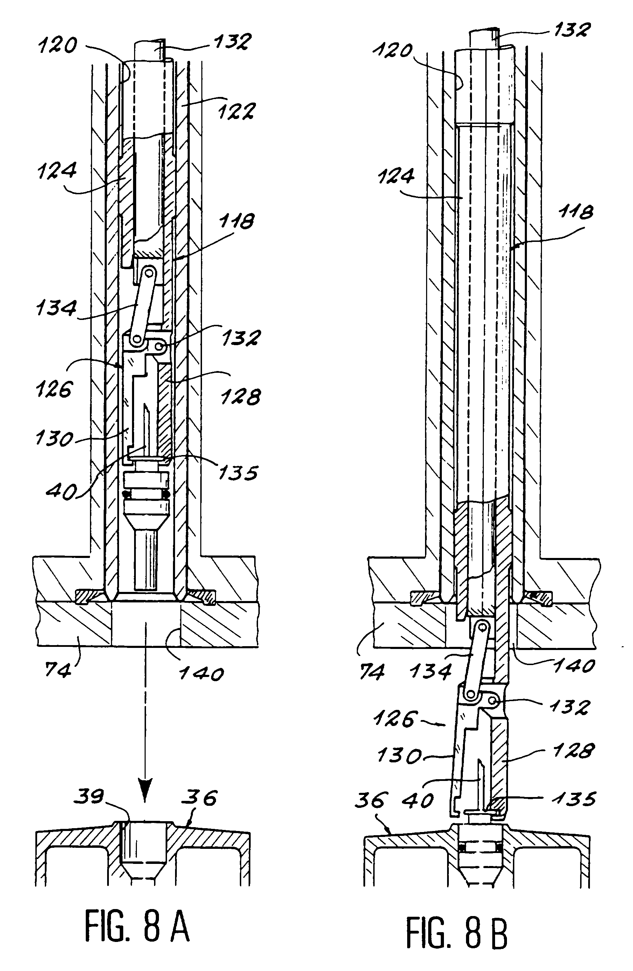

Le transfert de chaque cruchon sous vide entre le poste de distribution et l'installation de prélèvement selon l'invention est effectué pneumatiquement, le cruchon cheminant dans un tube par aspiration, sous l'action d'une centrale d'aspiration matérialisée sur les figures 1 et 6 par une pompe 46 et située à l'entrée de l'installation de prélèvement selon l'invention.

De façon plus précise, la pompe 46 est placée sur une canalisation 48, branchée en dérivation, immédiatement au-dessus d'un barillet pneumatique 52 sur un tube de transfert 50, par lequel les cruchons sont acheminés jusqu'à l'installation de prélèvement. Le barillet pneumatique 52 constitue un moyen d'isolement, qui est situé au-dessus du bouchon tournant 20, dans l'axe de ce dernier. Un tube souple 54 relie le barillet 52 à l'extrémité supérieure d'un conduit de transfert vertical 60 qui traverse le bouchon tournant 20. Le caractère souple du tube 54 permet de tenir compte des déplacements du conduit 60 de transfert des cruchons traversant le bouchon tournant 20, par rapport au barillet pneumatique 52.

Comme indiqué précédemment, le barillet pneumatique 52 constitue un moyen d'isolement permettant, lorsqu'il occupe une première position d'isolement, d'isoler l'un par rapport à l'autre le tube de transfert 50 et le tube souple 54.

Le barillet pneumatique 52 peut également occuper une position de transfert ou de passage, dans laquelle le tube de transfert 50 et le tube souple 54 communiquent entre eux.

Pour cela, le barillet pneumatique ou le moyen d'isolement correspondant peut être réalisé de différentes manières. A titre d'exemple, il peut s'agir d'un barillet comportant un boisseau rotatif dans lequel est formé un passage qui n'est pas aligné avec le tube de transfert 50 dans la position d'isolement du barillet, ce passage mettant en communication le tube de transfert 50 et le tube souple 54 dans la position de passage du barillet.

Comme l'illustre très schématiquement la figure 1, le barillet pneumatique 52, ainsi que la pompe 46 et les installations annexes qui seront décrites ultérieurement sont avantageusement montés sur une platine horizontale 56 placée au-dessus du bouchon tournant 20 et reliée à la dalle 12, à proximité de ce bouchon, par une potence 58.

Comme l'illustre plus précisément la figure 2, le conduit 60 de transfert des cruchons 44, sur lequel est raccordée l'extrémité inférieure du tube souple 54, traverse selon une direction verticale un élément cylindrique 62 de la dalle 12. De façon plus précise, cet élément cylindrique 62 est placé dans une ouverture circulaire 64 formée dans le bouchon tournant 20, de façon à pouvoir se déplacer verticalement entre deux positions extrèmes sans rupture du confinement.

Il est à noter que l'axe de l'élément cylindrique 62 ainsi que l'axe du conduit de transfert sont placés à une même distance de l'axe vertical du bouchon tournant 20 que le pion de confinement 42. Par conséquent, la rotation du bouchon tournant 20 a pour effet de faire décrire aux axes de l'élément cylindrique 62 et du conduit de transfert 60 un arc de cercle situé à égale distance des arcs de cercle sur lesquels sont réparties les aiguilles 40. En outre, cette distance séparant l'arc de cercle décrit par l'axe de l'élément cylindrique 62 des arcs de cercle sur lesquels se trouvent les aiguilles 40 est égale à la distance qui sépare l'axe du conduit de transfert vertical 60 de l'axe de l'élément cylindrique 62.

Les deux positions extrêmes que peut occuper l'élément 62 correspondent respectivement à une position haute d'attente, dans laquelle la face inférieure de l'élément cylindrique affleure la face inférieure du bouchon tournant 20, et une position basse de prélèvement, permettant notamment de piquer le bouchon d'un cruchon sur l'une des aiguilles 40 pour assurer le prélèvement, comme on le verra ultérieurement.

Le déplacement de l'élément cylindrique 62 entre ces deux positions est commandé par un moteur à courant continu 66 illustré sur la figure 1. Le corps de ce moteur 66 est monté sur un support 68 relié par des colonnettes verticales 70 à une bride faisant saillie vers le haut sur le bouchon tournant 20 autour de l'élément cylindrique 62.

Un capteur différentiel à déplacement linéaire (non représenté) contrôle la position de l'élément cylindrique 62. La position basse de cet élément est en outre détectée par un capteur inductif (non représenté).

l'élément cylindrique 62 occupe normalement sa position haute d'attente et sa descente n'est possible que lorsque le bouchon tournant 20 est verrouillé par le verrou 33 dans une position angulaire déterminée.

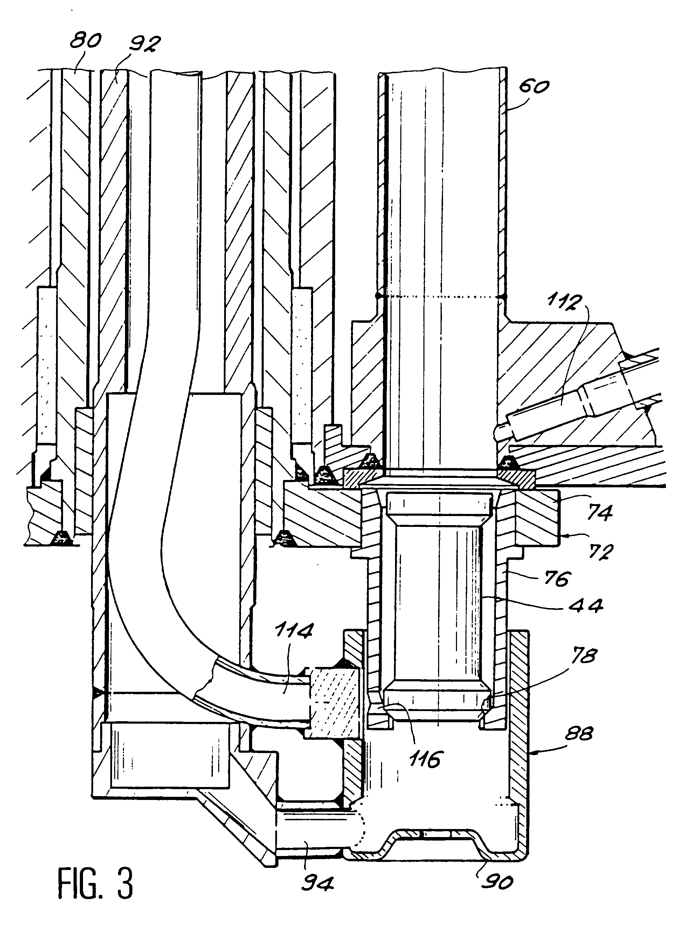

Comme l'illustrent notamment les figures 2 et 3, l'élément cylindrique 62 supporte de façon rotative un barillet porte-cruchon 72 situé en dessous de cet élément et dont l'axe de rotation est confondu avec l'axe vertical de ce dernier.

Le barillet porte-cruchon 72 comporte principalement un disque horizontal 74, situé immédiatement en dessous de l'élément cylindrique, et une nacelle 76 de réception d'un cruchon, présentant généralement la forme d'un tube qui traverse le disque 74 et fait saillie vers le bas à partir de ce dernier. Le diamètre intérieur de la nacelle 76 est identique à celui du conduit de transfert 60 et elle présente à son extrémité inférieure un épaulement 78 sur lequel peut venir en appui un cruchon 44, lorsque la nacelle 76 est placée dans l'alignement du conduit de transfert 60 comme l'illustre la figure 3. La longueur de la nacelle 76 est telle que, lorsqu'un cruchon 44 est en appui contre l'épaulement 78, l'extrémité supérieure du cruchon est située légèrement en dessous du plan supérieur du disque 74, de telle sorte que la rotation du barillet 72 est possible.

Le barillet rotatif 72 comporte en outre un arbre de commande creux 80, qui traverse selon son axe l'élément cylindrique 62, de façon à assurer le supportage en rotation du barillet autour de cet axe par l'élément cylindrique 62.

L'arbre de commande 80 permet également d'assurer la commande de rotation du barillet par un moteur à courant continu 81 monté au-dessus de l'élément cylindrique 62 et dont l'arbre de sortie vertical 82 entraîne en rotation un pignon 84 qui s'engrène sur une roue dentée 86 formée à l'extrémité supérieure de l'arbre de commande 80.

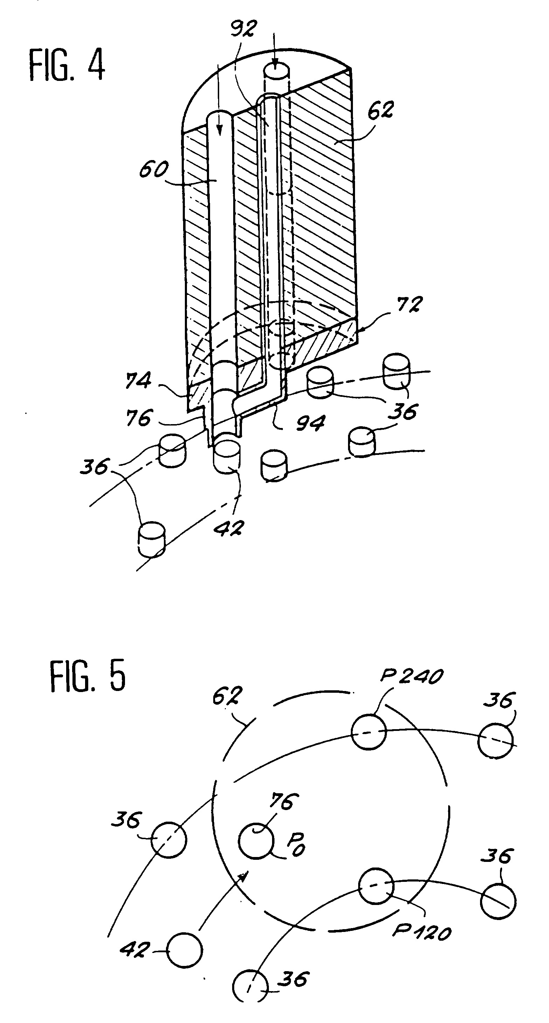

Au cours de sa rotation, le barillet 72 peut occuper trois positions présélectionnées disposées à 120° les unes des autres. Une première position Pₒ (figure 5), illustrée notamment sur les figures 2 et 3, correspond à l'alignement de la nacelle 76 avec le conduit de transfert 60. Cette position, qui correspond à l'arrivée ou au départ d'un cruchon 44, permet également par une rotation du bouchon tournant 20, puis une descente de l'élément cylindrique 62, d'amener l'extrémité inférieure de la nacelle 76 en appui sur le pion de confinement 42 afin d'assurer un confinement entre le volume clos 18 délimité par la cuve 14 et le conduit de transfert 60.

Les deux autres positions présélectionnées P₁₂₀ et P₂₄₀ (figure 5) du barillet 72 permettent quant à elles d'amener l'axe de la nacelle 76 au-dessus de l'un ou l'autre des deux arcs de cercle sur lesquels sont réparties les aiguilles 40. Ces positions permettent donc d'amener un cruchon 44 logé dans la nacelle 76 au-dessus de l'une quelconque des aiguilles 40, par une rotation du bouchon tournant 20, afin d'effectuer les prélèvements désirés.

La position du barillet 72 est donnée en continu par une mesure angulaire obtenue à l'aide d'un capteur à déplacement linéaire (non représenté), manoeuvré par une came solidaire du barillet.

L'installation de prélèvement d'échantillons selon l'invention comprend de plus un organe anti-arrachement 88, représenté en détail sur la figure 3, qui a pour fonction de maintenir le corps de l'aiguille 40 emboîté dans le réceptacle 39 du pot de prélèvement 38, lorsqu'un cruchon 44 venant d'être piqué sur l'aiguille afin d'effectuer un prélèvement est déplacé vers le haut pour dégager complètement le cruchon de l'aiguille correspondante.

Cet organe anti-arrachement 88 a la forme d'un manchon entourant la partie de la nacelle 76 qui fait saillie en dessous de la plaque 74. Ce manchon est fermé à sa base par une plaque 90 percée en son centre pour permettre le passage des aiguilles 40 et aussi de façon à pouvoir être emboîtée sur le pion de confinement 42.

L'organe anti-arrachement 88 est monté sur la nacelle 76 de façon à pouvoir se déplacer verticalement entre une position haute, dans laquelle la plaque 90 affleure l'extrémité inférieure de la nacelle et une position basse, illustrée sur la figure 3, dans laquelle cette plaque 90 est située à une distance déterminée en dessous de l'extrémité inférieure de la nacelle.

Le supportage de l'organe anti-arrachement 88, permettant son déplacement entre ces deux positions, est assuré par un tube rigide 92 traversant l'élément cylindrique 62 à l'intérieur de l'arbre de commande creux 80 et dont l'extrémité inférieure porte l'organe 88 par l'intermédiaire d'un tube de liaison 94 débouchant dans l'organe anti-arrachement 88 juste au-dessus de la plaque 90.

Des moyens élastiques tels qu'un ressort 96 (figure 2) sont interposés entre l'extrémité supérieure de l'arbre de commande creux 80 et l'extrémité supérieure du tube rigide 92, au-dessus de l'élément cylindrique 62, de façon à maintenir normalement l'organe anti-arrachement 88 dans sa position basse par rapport à la nacelle 76.

En plus de leur fonction de supportage vis-à-vis de l'organe anti-arrachement 88, le tube rigide 92 et le tube de liaison 94 constituent un circuit pneumatique fermé, raccordé par l'extrémité supérieure du tube rigide 92 à une canalisation d'amenée d'air 98 qui apparaît notamment sur les figures 1 et 6. Cette canalisation d'amenée d'air 98 communique par une première élextrovanne 100 avec des moyens de ventilation de la cuve 14 et, par une deuxième électrovanne 102, avec des moyens d'amenée d'air comprimé servant, comme on le verra par la suite, à faire remonter jusqu'au barillet pneumatique 52 un cruchon dans lequel un prélèvement a été effectué.

Dans les conditions normales de fonctionnement de l'installation, l'électrovanne 100 est ouverte afin d'assurer la ventilation de la cuve 14. Au contraire, l'électrovanne 102 est fermée. La canalisation d'amenée d'air 98 et le tube rigide 92 constituent ainsi, lorsqu'ils sont reliés par l'électrovanne 102 à des moyens d'amenée d'air comprimé, une partie des moyens de transfert pneumatique des cruchons au travers de la dalle 12.

Ces moyens de transfert pneumatique des cruchons comprennent également des moyens pour favoriser la descente des cruchons dans la nacelle 76, qui s'effectue normalement par gravité. Ces moyens comprennent une deuxième canalisation d'amenée d'air 104 (figure 6), qui communique avec des moyens d'amenée d'air comprimé au travers d'une troisième électrovanne 106. Cette canalisation d'amenée d'air 104 débouche dans le tube de transfert 50, juste au-dessus du barillet pneumatique 52. Après ouverture de l'électrovanne 106 et lorsque le barillet pneumatique 52 met en communication le tube de transfert 50 avec le tube souple 54, ces moyens permettent d'assister pneumatiquement la descente de chaque cruchon depuis le barillet 52 jusque dans la nacelle 76, qui se trouve alors alignée avec le conduit de transfert 60.

L'installation de prélèvement d'échantillons conforme à l'invention comporte de plus un certain nombre de détecteurs permettant de connaître à tout instant la position exacte occupée par un cruchon à l'intérieur de l'installation.

Ces détecteurs comportent notamment un détecteur 108 (figure 6) de passage d'un cruchon dans le tube de transfert 50, détectant l'arrivée d'un cruchon au niveau du barillet pneumatique 52. Ce détecteur 108, ainsi qu'un deuxième détecteur 110 placé dans le tube souple 54 immédiatement en dessous du barillet pneumatique 52, peuvent notamment être constitués par des détecteurs à fibre optique.

Comme l'illustre plus précisément la figure 3, un troisième détecteur à fibre optique 112 est monté dans l'élément cylindrique 62, dans le bas du conduit de transfert 60, pour détecter le passage d'un cruchon 44 et la non présence d'un cruchon dans l'élément cylindrique lorsqu'un prélèvement est effectué. Ce détecteur 112 permet d'éviter qu'un cruchon 44 ne soit cisaillé par une rotation du barillet 72.

Enfin, un quatrième détecteur à fibre optique 114 dont la fibre optique chemine à l'intérieur du tube rigide 92, est placé sur l'organe anti-arrachement 88 et se trouve en face d'un trou 116 formé dans la nacelle 76, au niveau de l'épaulement 78, lorsque l'organe anti-arrachement 88 est en position basse par rapport à la nacelle comme l'illustre la figure 3. Ce détecteur 112 permet de s'assurer de la présence d'un cruchon 44 dans la nacelle 76 avant qu'un prélèvement ne soit effectué.

L'installation de prélèvement d'échantillons fluides qui vient d'être décrite en se référant aux figures 1 à 6, fonctionne de la façon suivante.

Lorsqu'aucun prélèvement n'est en cours, le barillet pneumatique 52 est dans sa position d'isolement et la cuve 14 est ventilée par de l'air injecté au travers de l'électrovanne 100 par la canalisation d'amenée d'air 98 et le tube rigide 92. Par ailleurs, l'élément cylindrique 62 est en position haute, la nacelle 76 alignée avec le conduit de transfert 60 et le bouchon tournant 20 indexé dans sa position angulaire pour laquelle le conduit de transfert 60 et la nacelle 76 se trouvent situés au-dessus du pion de confinement 42.

Lorsqu'un prélèvement doit être effectué, un cruchon sous vide est expédié depuis le poste de distribution (non représenté) jusqu'au barillet pneumatique 52 par le tube de transfert 50, sous l'action de la pompe 46. Lorsqu'il arrive au-dessus du barillet 52, le cruchon est détecté par le détecteur 108. La centrale d'aspiration matérialisée par la pompe 46 est alors arrêtée.

L'électrovanne 100 assurant la ventilation de la cuve 14 est ensuite fermée. Le barillet pneumatique 52 est alors actionné afin de mettre en communication le tube de transfert 50 et le tube souple 54. Simultanément, l'électrovanne 106 est ouverte afin d'assurer une entrée d'air sous pression au-dessus du cruchon sous vide précédemment stoppé par le barillet pneumatique 52.

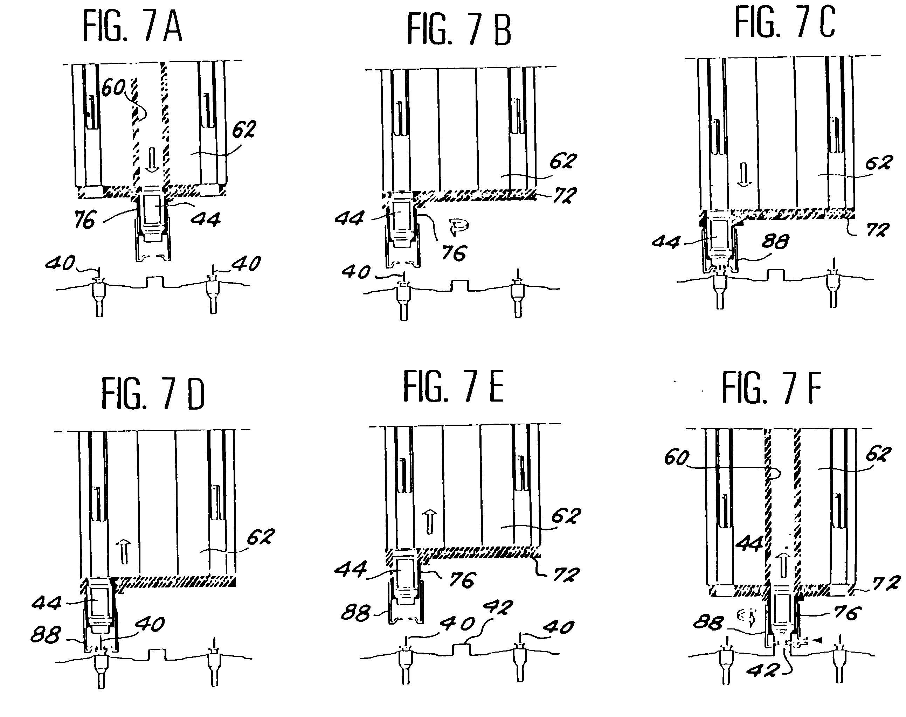

L'action conjointe de l'air comprimé admis par l'électrovanne 106, de la gravité et de la dépression régnant dans la cuve 14 ont pour effet de faire descendre le cruchon dans le tube souple 54 puis dans le conduit de transfert 60 jusqu'à l'intérieur de la nacelle 76 comme illustré sur la figure 7A. Le passage du cruchon est détecté successivement par le capteur 110, le capteur 112 puis le capteur 114. L'électrovanne 106 est alors refermée et le barillet pneumatique 52 ramené dans sa position d'isolement.

Selon l'arc de cercle sur lequel se trouve l'aiguille 40 correspondant au prélèvement à effectuer, le barillet 72 tourne alors de 120° ou 240°. Il passe ainsi de la position P₀ à une position P₁₂₀ ou P₂₄₀, comme l'illustre schématiquement la figure 5.

Afin d'amener la nacelle 76 portant le cruchon 44 sous vide au-dessus de l'aiguille 40 sur laquelle un prélèvement doit être effectué, on fait aussi tourner le bouchon tournant 20 jusqu'à ce que l'axe de la nacelle 76 se trouve à la verticale de cette aiguille. Le cruchon 44 contenu dans la nacelle 76 passe ainsi dans une position de sélection de l'aiguille considérée illustrée sur la figure 7B.

L'élément cylindrique 62 se déplace alors vers le bas, entraînant avec lui le barillet 72 et l'organe anti-arrachement 88. Après que ce dernier soit venu coiffer le corps de l'aiguille 40 correspondante, la descente de l'élément cylindrique 62 et du barillet 72 se poursuit, de telle sorte que l'aiguille 40 perfore le bouchon de caoutchouc obturant l'extrémité inférieure du cruchon 44. Il est à noter que ce dernier est alors maintenu à l'encontre de tout déplacement vers le haut par son appui contre la face inférieure de l'élément cylindrique 62, puisque la nacelle 76 est alors décalée angulairement du conduit de transfert 60. Dans ces conditions, illustrées sur la figure 7C, un prélèvement est réalisé automatiquement sous l'effet d'aspiration créé par le vide présent à l'intérieur du cruchon 44.

Lorsque le temps nécessaire au prélèvement est écoulé, l'élément cylindrique 62 remonte, entraînant avec lui la nacelle 76 dans laquelle se trouve le cruchon 44 qui contient le prélèvement. Au début de ce mouvement et tant que l'aiguille 40 reste piquée dans le bouchon du cruchon 44, l'organe anti-arrachement 88 reste plaqué contre le corps de l'aiguille 40 sous l'action du ressort , 96 (figure 2). Cette caractéristique, illustrée sur la figure 7D, permet d'éviter que l'aiguille 40 ne soit arrachée de son pot de prélèvement sous l'effet de la remontée du cruchon.

Ensuite et comme l'illustre la figure 7E, l'organe anti-arrachement 88 remonte avec l'élément cylindrique 62 et la nacelle 76 contenant le cruchon 44.

Le prélèvement est alors terminé et le cruchon peut être expédié vers un laboratoire d'analyses. Pour cela, il est évacué de l'installation par le même chemin qu'il y est entré. A cet effet, le barillet 72 est ramené par une nouvelle rotation de sa position P₁₂₀ ou P₂₄₀ à sa position initiale P₀ et une nouvelle rotation du bouchon tournant 20 permet d'amener le conduit de transfert 60 et la nacelle 76 contenant le cruchon à la verticale du pion de confinement 42, qui détermine, comme on l'a vu la position dans laquelle s'effectue l'évacuation des cruchons.

Lorsque cette position est atteinte, l'élément cylindrique 62 est descendu afin que le trou formé dans la plaque 90 de l'organe anti-arrachement 88 soit obturé par le pion de confinement 42. Le volume 18 intérieur à la cuve 14 est alors isolé du conduit de transfert 60, de telle sorte que le barillet pneumatique 52 peut être amené dans sa position de passage. L'électrovanne 102 est ensuite ouverte, ce qui a pour effet d'injecter de l'air comprimé en dessous du cruchon contenu dans la nacelle 76, par la canalisation 98, le tube rigide 92 et le tube de liaison 94, comme illustré sur la figure 7F. Cet air comprimé commande la remontée du cruchon 44, contenant l'échantillon prélevé, dans le conduit de transfert 60, dans le tube souple 54, puis dans le tube de transfert 50 qui communique alors avec le tube 54 au travers du barillet pneumatique 52. Le cruchon est ensuite acheminé jusqu'à un laboratoire d'analyses approprié par aspiration, à l'aide d'une installation d'aspiration associée à ce laboratoire et comparable à celle qui est illustrée par la pompe 46 sur la figure 1.

Lorsque le cruchon contenant le prélèvement arrive dans le tube de transfert 50, le barillet pneumatique 52 revient dans sa position d'isolement, l'électrovanne 102 se referme et les autres éléments de l'installation reviennent dans leur état initial décrit précédemment.

L'installation de prélèvement d'échantillons fluides dont la structure et le fonctionnement viennent d'être décrits est avantageusement équipée en outre de moyens permettant de démonter les aiguilles 40 obturées ou endommagées et de les remplacer par des aiguilles neuves.

Comme l'illustrent en particulier les figures 7A et 78, ces moyens comprennent essentiellement deux outils de préhension qui sont montés verticalement dans l'élément cylindrique 62, en des emplacements espacés angulairement respectivement de 120° et de 240° par rapport à l'axe du conduit de transfert 60, autour de l'axe de l'élément cylindrique 62, et à une même distance de l'axe de l'élément 62 que l'axe du conduit de transfert 60.

Chacun de ces outils de préhension 118 est logé dans une alvéole cylindrique 120, d'axe vertical, formée à l'intérieur d'un fourreau porte-outil 122. Il est à noter que le diamètre de chacune des alvéoles 120 est inférieur au diamètre du conduit de transfert 60, de telle sorte que l'extrémité inférieure de chacun des fourreaux porte-outil 122 sert de butée au cruchon 44 placé dans la nacelle, lors de l'opération de prélèvement décrite précédemment, au cours de laquelle les cruchons se trouvent situés en dessous de l'une ou l'autre des alvéoles 120.

Chacun des outils de préhension 118 comporte un corps cylindrique 124, mobile verticalement dans l'alvéole 120 correspondante, ce corps cylindrique 124 supportant une pince 126 à son extrémité inférieure. Cette pince 126 se compose d'une mâchoire fixe 128, solidaire du corps 124, et d'une mâchoire mobile 130 articulée sur la mâchoire fixe 128 par un axe 132 orthogonal à l'axe longitudinal de l'alvéole 120.

La commande du pivotement de la mâchoire mobile 130 de la pince est assurée par une tige 132 qui coulisse selon l'axe de l'alvéole 120 à l'intérieur du corps cylindrique 124 et dont l'extrémité inférieure est reliée à la mâchoire mobile 130 par une biellette articulée 134. Le déplacement de la tige 132 vers le bas, correspondant à la fermeture de la pince 126, est assuré par un électro-aimant (non représenté) interposé entre cette tige et le corps cylindrique 124.

Le déplacement de l'outil de préhension 118 dans son ensemble à l'intérieur de l'alvéole 120 est commandé quant à lui par un moteur à courant continu (non représenté), sous le contrôle d'un capteur à déplacement linéaire (non représenté).

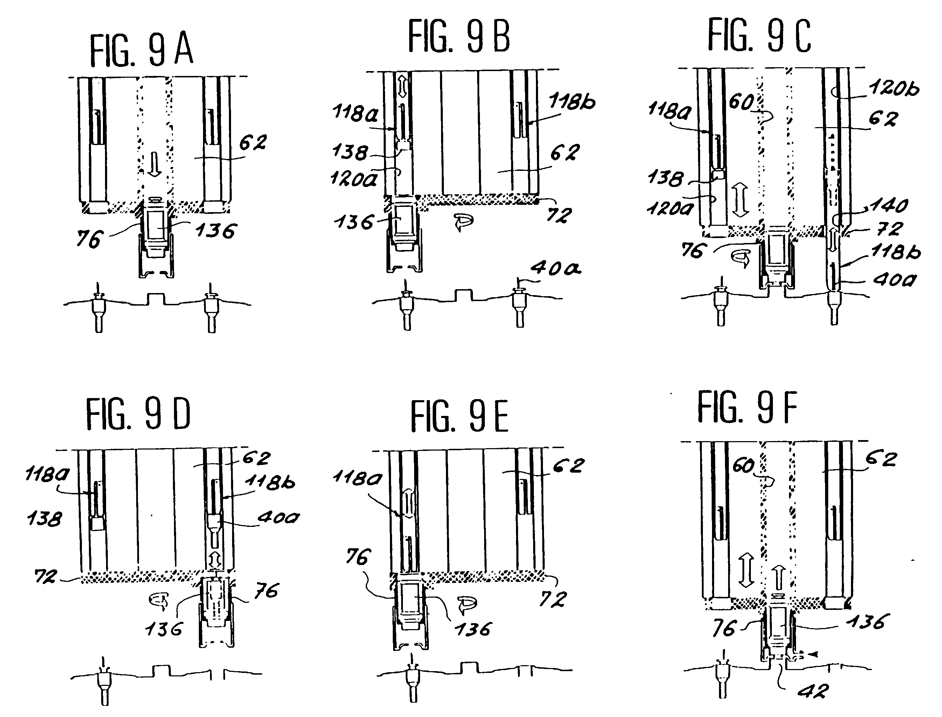

Les caractéristiques des deux outils de préhension 118, qui sont identiques, sont conçues de façon à permettre la préhension par chacun d'entre eux d'une aiguille 40 (figures 8A et 8B) ou du bouchon d'un conteneur de forme générale analogue à celle des cruchons 44 et servant à transporter les aiguilles 44 neuves et usagées. A cet effet, les extrémités des mâchoires 128 et 130 présentent la forme de crochets aptes à venir se placer sur des collerettes 135 formées sur le corps des aiguilles 40 et sur le bouchon des conteneurs de transport des aiguilles.

Un capteur à déplacement linéaire unique (non 5 représenté) commande l'arrêt de l'outil de préhension 118 lors de son déplacement vers le bas préalable à la prise ou à la pose d'un bouchon ou d'une aiguille.

Comme l'illustrent les figures 7A et 7B, le disque 74 du barillet 72 comporte deux passages 140 qui sont alignés avec les alvéoles 120 lorsque la nacelle 76 est alignée avec le conduit de transfert 60 et dont le diamètre est légèrement supérieur à celui des alvéoles.

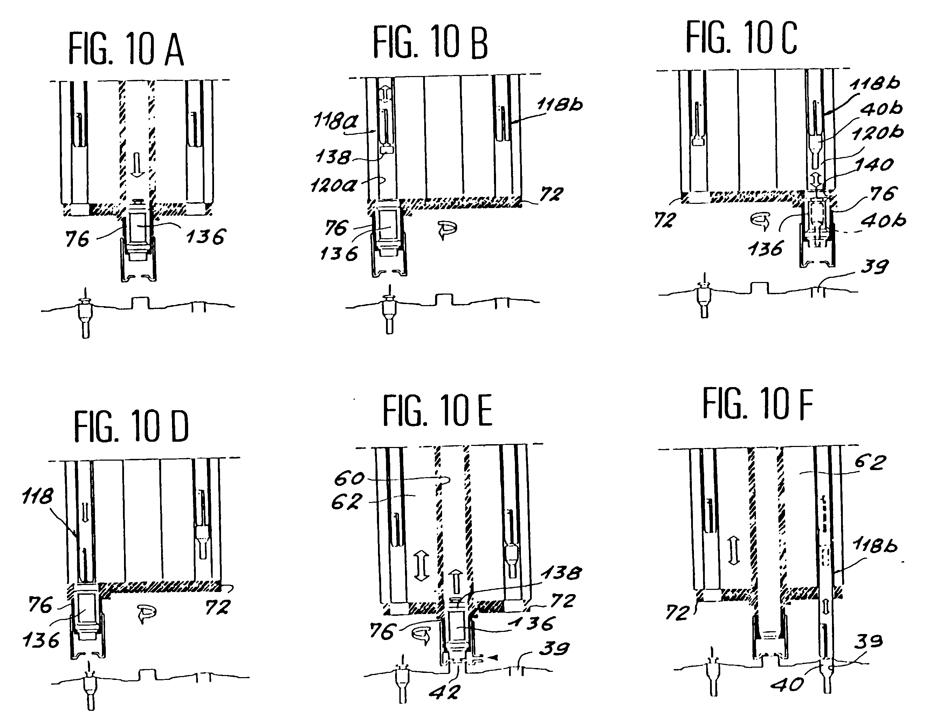

Grâce aux deux outils de préhension 118 implantés dans l'élément cylindrique 62 du bouchon tournant, il est possible, par deux opérations successives commandées intégralement à distance, de démonter une aiguille usagée, puis de la remplacer par une aiguille neuve, comme on va à présent le décrire en se référant aux figures 9A à 9F puis aux figures 10A à 10F. Dans cette description, afin de les distinguer malgré leur identité, les références des deux outils de préhension et des alvéoles correspondantes seront affectées des lettres a et b.

Dans un premier temps, comme illustré schématiquement sur la figure 9A, un conteneur vide 136 apte à contenir une aiguille est introduit dans la nacelle 76 d'une manière analogue à l'introduction d'un cruchon 44 dans cette même nacelle, décrite précédemment.

Une rotation de 120° ou de 240° du barillet 72 permet ensuite d'amener la nacelle 76 en dessous de l'outil de préhension 118a dont l'axe est aligné avec l'arc de cercle ne comportant pas l'aiguille 40a à remplacer. Une rotation du bouchon tournant 20 permet par ailleurs d'amener l'axe de l'autre outil 118b à la verticale de l'aiguille 40a à remplacer. L'outil 118a est alors descendu et sa pince est actionnée, de façon à saisir le bouchon 138 du conteneur 136, puis à escamoter ce bouchon à l'intérieur de l'alvéole 120a contenant l'outil de préhension 118a. Cette étape est illustrée de façon schématique par la figure 9B.

Par une nouvelle rotation du barillet 72, on ramène la nacelle 76 dans sa position initiale d'alignement avec le conduit de transfert 60. Cette rotation a pour effet d'amener les deux passages 140 formés dans le plateau 74 du barillet 72 en dessous de chacune des alvéoles 120a et 120b. L'outil de préhension 118b est alors descendu et sa pince 126 actionnée de façon à saisir l'aiguille 40a, puis à l'escamoter à l'intérieur de l'alvéole 120b correspondante. Cet ensemble d'opérations est illustré schématiquement sur la figure 9C.

Comme le montre la figure 9D, une nouvelle rotation du barillet 72 amène ensuite la nacelle 76 dans laquelle se trouve toujours le conteneur 156 dépourvu de son bouchon en dessous de l'outil de préhension 118b portant l'aiguille 40a à remplacer. Une nouvelle descente de cet outil 118b, puis un nouvel actionnement de sa pince, permettent de placer l'aiguille 40a à remplacer dans le conteneur 136.

Après remontée de l'outil de préhension 118b, le barillet 72 subit une nouvelle rotation, illustrée sur la figure 9E, qui a pour effet d'amener la nacelle 76 dans laquelle se trouve le conteneur 136, qui contient alors l'aiguille 40a, à la verticale de l'outil de préhension 118a qui porte toujours le bouchon du conteneur. Ce dernier est alors obturé en faisant descendre l'outil de préhension 118a et en libérant le bouchon par un actionnement de la pince de cet outil.

L'outil 118a est à son tour escamoté dans l'élément cylindrique 62 et une nouvelle rotation combinée du barillet 72 et du bouchon tournant 20 permet d'aligner la nacelle 76, le conduit de transfert 60 et le pion de confinement 42. Dans ces conditions, le conteneur 136 contenant l'aiguille usagée 40a peut être évacué, de la même manière qu'un cruchon 44, par une descente de l'élément cylindrique 62 ayant pour effet d'isoler le conduit de transfert 60 du volume intérieur de la cuve 14 à l'aide du pion de confinement 42, puis par une injection d'air comprimé en dessous de la nacelle 76, commandée par une ouverture de l'électrovanne 102 (figure 9F).

La mise en place d'une aiguille neuve 40b peut ensuite être envisagée, de la manière illustrée schématiquement sur les figures 10A à 10F.

Dans un premier temps, un conteneur 136 transportant une aiguille neuve 40 est introduit dans la nacelle 76 de la manière décrite précédemment pour les cruchons 44. Cette première étape est illustrée sur la figure 10A.

Ensuite et comme l'illustre la figure 10B, le barillet 72 est entraîné en rotation afin d'amener la nacelle 76 en face de l'outil de préhension 118a qui ne se trouve pas sur l'arc de cercle comportant le réceptacle 39 de la tête de prélèvement dépourvue d'aiguille. L'outil de préhension 118a est alors actionné pour venir saisir le bouchon 138 du conteneur 136 et l'escamoter dans l'alvéole 120a correspondante.

Une nouvelle rotation du barillet 72, illustrée sur la figure 10C, permet d'amener la nacelle 76 dans laquelle se trouve le conteneur ouvert 136, contenant l'aiguille neuve 40b, à la verticale du deuxième outil de préhension 118b. Ce dernier est alors actionné à son tour de façon à venir saisir l'aiguille neuve 40b et à l'escamoter dans l'alvéole 120b correspondante, au travers du passage 140.

Comme l'illustre la figure 10D, le barillet 72 subit une nouvelle rotation ayant pour effet de ramener la nacelle 76 à la verticale de l'outil de préhension 118a portant le bouchon 138. Ce dernier peut ainsi être remis en place sur le conteneur 136 qui est alors vide.

Ce conteneur vide 136 est ensuite évacué, après une nouvelle rotation du barillet 72 amanant la nacelle 76 contenant le conteneur vide, fermé par son bouchon 138, dans le prolongement du conduit de transfert 60, qui se trouve alors à la verticale du pion de confinement 42. L'évacuation du conteneur est réalisée de la même manière que précédemment, en descendant l'élément cylindrique 62 de façon à isoler le volume intérieur de la cuve 14 du conduit de transfert 60 à l'aide du pion de confinement 42, comme illustré sur la figure 10E, par de l'air comprimé injecté en dessous de la nacelle 76 par les tubes 92 et 94, en ouvrant l'électrovanne 102.

L'élément cylindrique 62 est ensuite remonté et l'outil de préhension 118b portant l'aiguille neuve 40b est amené à la verticale du réceptacle 39 de la tête de préhension dépourvue d'aiguille, par une rotation du bouchon tournant 20. L'aiguille neuve 40b peut alors être introduite dans ce réceptacle par la descente combinée de l'élément cylindrique 62 et de l'outil de préhension 118b portant l'aiguille. Cette étape est illustrée schématiquement sur la figure 10F. Lorsqu'elle est terminée, les différents organes de l'installation selon l'invention sont ramenés dans leur état initial décrit précédemment.

La figure 10B illustre également, plus en détail, l'introduction de l'aiguille neuve 40b dans le réceptacle 39 de la tête de prélèvement correspondante, à l'aide de l'outil de préhension 118.

Bien entendu, l'invention n'est pas limitée au mode de réalisation qui vient d'être décrit à titre d'exemple, mais en couvre toutes les variantes. On comprendra notamment que, dans le cas particulier où la cuve 14 ne contiendrait qu'une ou deux têtes de prélèvement, le bouchon tournant 20 peut être supprimé.

## Revendications

1. Installation de prélèvement d'échantillons fluides dans une zone confinée (10) située sous une dalle de protection (12), cette installation comprenant une cuve de prélèvement (14) située sous la dalle (12), au moins une aiguille de prélèvement (40) traversant un fond de la cuve, et des moyens de manutention à distance pour transférer un à un des cruchons de prélèvement (44) dans et hors de la cuve (14), au travers de la dalle (12), et pour piquer ces cruchons (44) un à un sur ladite aiguille (40), afin d'effectuer un prélèvement, caractérisée par le fait que lesdits moyens de manutention à distance comprennent :
- un conduit (60) de transfert des cruchons (44), traversant un élément (62) de la dalle (12), mobile verticalement entre une position haute d'attente et une position basse de prélèvement ;
- un barillet (72) monté rotatif sous ledit élément (62) de dalle (12), autour d'un axe décalé par rapport audit conduit (60), et comportant une nacelle (76) de réception de cruchon (44) apte à être amenée, par rotation du barillet, dans une position angulaire de réception et d'évacuation de cruchon (44), dans laquelle la nacelle est placée sous le conduit (60), et dans au moins une position angulaire de prélèvement, dans laquelle la nacelle (76) peut être amenée au-dessus de l'aiguille de prélèvement (40) ; et
- des moyens (102, 106) de transfert pneumatique des cruchons (44), vers le bas et vers le haut, dans ledit conduit (60).

2. Installation selon la revendication 1, caractérisée par le fait que les moyens de manutention à distance comprennent plusieurs aiguilles de prélèvement (40) disposées selon au moins un arc de cercle d'axe décalé par rapport à l'axe du barillet (76),et un bouchon tournant (20) d'axe confondu avec l'axe de cet arc de cercle, formant au-dessus de la cuve (14) une partie de la dalle (12), et dans lequel est logé ledit élément de dalle (62), de telle sorte que, dans ladite position angulaire de prélèvement de la nacelle (76), cette dernière peut être amenée au-dessus de chacune des aiguilles par une rotation du bouchon tournant.

3. Installation selon l'une quelconque des revendications 1 et 2, caractérisée par le fait que les moyens de manutention à distance comprennent des moyens de commande (82) de rotation du barillet (72), montés sur ledit élément de dalle (62) et agissant sur un arbre de commande (80) traversant l'élément de dalle selon son axe et solidaire du barillet (72) à son extrémité inférieure.

4. Installation selon la revendication 3, caractérisée par le fait que les moyens de transfert pneumatique des cruchons (44) comprennent une canalisation (98) d'amenée d'air qui chemine dans ledit arbre de commande (80) et débouche en dessous de la nacelle (76).

5. Installation selon la revendication 4, caractérisée par le fait que ladite canalisation d'amenée d'air (98) communique avec des moyens de ventilation de la cuve (14) au travers de premiers moyens formant vanne (100) et avec des moyens d'amenée d'air comprimé au travers de deuxièmes moyens formant vanne (102).

6. Installation selon l'une quelconque des revendications précédentes, caractérisée par le fait que, chaque aiguille de prélèvement (40) étant emboîtée dans un réceptacle (38) prévu dans le fond de la cuve (14), les moyens de manutention à distance comprennent un organe anti-arrachement (88) monté sur la nacelle (76) de façon à pouvoir se déplacer sur celle-ci entre une position haute dans laquelle cet organe (88) affleure l'extrémité inférieure de la nacelle (76) et une position basse dans laquelle cet organe (88) est espacé de l'extrémité inférieure de la nacelle (76) d'une distance sensiblement égale à la longueur d'une partie de chaque aiguille (40) faisant saillie au-dessus du réceptacle.

7. Installation selon la revendication 6, caractérisée par le fait que l'organe anti-arrachement (88) est sollicité vers sa position basse par des moyens élastiques (96).

8. Installation selon la revendication 6, combinée avec l'une quelconque des revendications 4 et 5, caractérisée par le fait que la canalisation (98) d'amenée d'air comporte un tube rigide (92), logé de façon coulissante dans l'arbre de commande (80) et portant l'organe anti-arrachement (88) à son extrémité inférieure.

9. Installation selon l'une quelconque des revendications 6 à 8, caractérisée par le fait que ledit élément de dalle (62) comporte deux alvéoles (120) dont l'une au moins peut être amenée au-dessus d'une aiguille (40) et sous chacune desquelles peut être amenée la nacelle (76), par une rotation du barillet (72), un outil de préhension (118) étant logé de façon coulissante dans chacune des alvéoles (120) et apte à saisir un bouchon de cruchon et une aiguille.

10. Installation selon la revendication 9, caractérisée par le fait que chacun des outils de préhension comprend une pince (126) montée à l'extrémité inférieure d'un corps (124) coulissant dans l'alvéole (120), cette pince (126) comportant au moins une mâchoire pivotante (130) dont un pivotement est commandé par une tige (132) apte à coulisser dans le corps (124).

11. Installation selon l'une quelconque des revendications précédentes, caractérisée par le fait que le fond de la cuve (14) porte un plot de confinement (42) apte à obturer un orifice de passage de l'aiguille (40) formé dans l'extrémité inférieure de la nacelle (76), lorsque ledit élément de dalle (62) est en position basse et lorsque la nacelle (76) est placée sous le conduit (60) de transfert des cruchons (44).

12. Installation selon l'une quelconque des revendications précédentes, caractérisée par le fait que les moyens de manutention à distance comprennent un détecteur (114) de présence d'un cruchon (44) dans la nacelle (76).

13. Installation selon l'une quelconque des revendications précédentes, caractérisée par le fait que les moyens de manutention à distance comprennent de plus, des moyens d'isolement (52) placés au-dessus de la dalle (12) et raccordés au conduit (60) de transfert des cruchons (44) par un tube souple (54), ces moyens d'isolement (52) pouvant occuper une position d'isolement, dans laquelle le tube souple (54) est isolé d'un tube de transfert (50) placé au-dessus des moyens d'isolement (52), et une position de passage dans laquelle le tube souple (54) et le tube de transfert (50) communiquent entre eux.

14. Installation selon la revendication 13, caractérisée par le fait que les moyens de manutention à distance comprennent un deuxième détecteur (108) de passage d'un cruchon (44) dans le tube de transfert (50), au-dessus des moyens d'isolement (52).

15. Installation selon l'une quelconque des revendications 13 et 14, caractérisée par le fait que les moyens de transfert pneumatique des cruchons (44) comprennent de plus une deuxième canalisation d'amenée d'air (104) qui communique avec des moyens d'amenée d'air comprimé au travers de troisièmes moyens formant vanne (106) et qui débouche dans le tube de transfert (50), immédiatement au-dessus des moyens d'isolement (52).

## Patentansprüche

1. Vorrichtung zur Entnahme von Flüssigkeitsproben in einem abgegrenzten Bereich (10), der sich unter einer Schutzplatte (12) befindet, wobei diese Vorrichtung einen Entnahmebehälter (14) besitzt, der sich unter der Platte (12) befindet, mindestens eine Entnahmenadel (40), die durch den Boden des Behälters verläuft, Beförderungsvorrichtungen zur Beförderung zu einem der Entnahmegefäß (44) innerhalb und außerhalb des Behälters (14) quer durch die Platte (12) und zum Verbinden dieser Gefäße (44) mit der genannten Nadel (40), um eine Probenahme zu realisieren, die dadurch gekennzeichnet ist, daß die genannten Beförderungsvorrichtungen folgende Teile umfassen:
- eine Leitung (60) zum Transport der Gefäße (44), die ein Element (62) der Platte (12) durchquert und in vertikaler Richtung zwischen einer oberen Warteposition und einer unteren Entnahmeposition hin und her bewegt werden kann;
- ein Sammelgefäß (72), das unter dem genannten Element (62) der Platte (12) um eine Achse drehbar montiert ist, die in bezug auf die genannten Leitung (60) verschoben ist, und ein Schiffchen (76) zur Aufnahme des Gefäßes (44), das durch eine Drehung des Sammelgefäßes in eine Winkelposition zur Aufnahme und Entleerung des Gefäßes (44) gebracht werden kann, in der das Schiffchen unter der Leitung (60) und in mindestens eine Entnahmewinkelstellung plaziert wird, in welcher das Schiffchen (76) über die Entnahmenadel (40) gebracht werden kann; und
- pneumatische Vorrichtungen (102, 106) zum Transport der Gefäße (44) nach oben und nach unten innerhalb der genannten Leitung (60).

2. Vorrichtung nach Anspruch 1, dadurch gekennzeichnet, daß die Beförderungsvorrichtungen mehrere Entnahmenadeln (40) besitzen, die in mindestens einem Kreisbogen um die Achse angeordnet sind, die in bezug auf die Achse des Sammelgefäßes (72) verschoben ist, und einen Stopfen, der sich um die Achse dreht, die mit der Achse dieses Kreisbogens zusammenläuft, und der über dem Behälter (14) einen Teil der Platte (12) bildet, in dem sich das genannte Element der Platte (62) befindet, so daß letztere in der genannten Entnahmewinkelstellung des Schiffchens (76) durch eine Drehung des drehbaren Stopfens über jede der Nadeln gebracht werden kann.

3. Vorrichtung nach einem der Ansprüche 1 und 2, dadurch gekennzeichnet, daß die Beförderungsvorrichtungen Antriebsvorrichtungen (82) zum Drehen des Sammelgefäßes (72) besitzen, die an dem genannten Element der Platte (62) montiert sind und über eine Antriebswelle (80) wirken, die das Plattenelement entlang seiner Achse durchquert und an ihrem unteren Ende mit dem Sammelgefäß fest verbunden ist.

4. Vorrichtung nach Anspruch 3, dadurch gekennzeichnet, daß die pneumatischen Transportvorrichtungen der Gefäße (44) eine Leitung (98) für die Luftzufuhr enthalten, die in der genannten Antriebswelle (80) verläuft unter dem Schiffchen (76) nach außen führt.

5. Vorrichtung nach Anspruch 4, dadurch gekennzeichnet, daß die genannte Luftzuführleitung (98) mit der Entlüftungsvorrichtung des Behälters (14) über erste Vorrichtungen, die ein Ventil (100) bilden, und mit Druckluftzuführvorrichtungen über zweite Vorrichtungen, die ein Ventil (102) bilden, in Verbindung steht.

6. Vorrichtung nach einem beliebigen der vorangegangenen Ansprüche, dadurch gekennzeichnet, daß, während jede Entnahmenadel (40) in ein Gefäß (38) eingelassen ist, das sich auf dem Boden des Behälters (14) befindet, die Beförderungsvorrichtungen ein Aushebeblockierteil (88) enthalten, das auf dem Schiffchen (76) in der Weise montiert ist, daß es auf dem Schiffchen zwischen einer oberen Position, in der sich dieses Teil (88) auf gleicher Höhe mit dem unteren Ende des Schiffchens (76) befindet, und einer unteren Position verschiebbar ist, in der sich dieses Teil (88) in einem Abstand von dem unteren Ende des Schiffchens(76) befindet, der annähemd der Länge eines Abschnitts jeder Nadel (40) entspricht, der über dem Gefäß herausragt.

7. Vorrichtung nach Anspruch 6, dadurch gekennzeichnet, daß das Aushebeblockierteil (88) durch elastische Vorrichtungen (96) in seine untere Position gedrückt wird.

8. Vorrichtung nach Anspruch 6 zusammen mit einem beliebigen der Ansprüche 4 und 5, dadurch gekennzeichnet, daß die Luftzuführleitung (98) ein starres Rohr (92) enthält, das auf der Antriebsachse (80) verschiebbar angebracht ist und an seinem unteren Ende das Aushebeblockierteil (88) trägt.

9. Vorrichtung nach einem beliebigen der Ansprüche 6 bis 8, dadurch gekennzeichnet, daß das genannte Plattenelement (62) zwei Mulden (120) enthält, von denen mindestens eine über eine Nadel (40) in Position gebracht werden kann, und unter die (beide Mulden) das Schiffchen (76) in Position gebracht werden kann, und zwar durch eine Drehung des Sammelgefäßes (72), wobei ein Greifwerkzeug (118) verschiebbar in jeder der Mulden (120) plaziert wird, das in der Lage ist, einen Stopfen des Gefäßes und eine Nadel zu greifen.

10. Vorrichtung nach Anspruch 9, dadurch gekennzeichnet, daß jedes der Greifwerkzeuge eine Zange (126) enthält, die am unteren Ende eines Teils (124) befestigt ist, das in der Mulde (120) gleitet, wobei diese Zange (126) mindestens eine kippbare Klemmbacke (130) hat, deren Kippbewegung durch eine Stange (132) angetrieben wird, welche in der Lage ist, in dem Teil (124) zu gleiten.

11. Vorrichtung nach einem beliebigen der vorangegangenen Ansprüche, dadurch gekennzeichnet, daß der Boden des Behälters (14) einen Begrenzungskontakt (42) hält, der in der Lage ist, eine Durchgangsöffnung der Nadel (40), die sich in dem unteren Ende des Schiffchens (76) befindet, zu versperren, während das genannte Plattenelement (62) sich in der unteren Position befindet und das Schiffchen (76) unter der Leitung (60) zum Transport der Gefäße (44) plaziert ist.

12. Vorrichtung nach einem beliebigen der vorangegangenen Ansprüche, dadurch gekennzeichnet, daß Beförderungsvorrichtungen einen Detektor (114) zur Ermittlung der Anwesenheit eines Gefäßes (44) in dem Schiffchen (76) besitzen.

13. Vorrichtung nach einem beliebigen der vorangegangenen Ansprüche, dadurch gekennzeichnet, daß die Beförderungsvorrichtungen des weiteren Isoliervorrichtungen (52) besitzen, die sich über der Platte (12) befinden und an die Behältertransportleitung (60) durch ein Schlauchstück (54) angeschlossen sind, wobei diese Isoliervorrichtungen (52) eine Isolierposition einnehmen können, in der das Schlauchstück (54) von einem Transportrohr (50) getrennt ist, das über den Isoliervorrichtungen(52) abgebracht ist, und eine Durchgangsposition einnehmen kann, in der das Schlauchstück (54) und das Transportrohr (50) miteinander in Verbindung stehen.

14. Vorrichtung nach Anspruch 13, dadurch gekennzeichnet, daß die Beförderungsvorrichtungen einen zweiten Durchgangsdetektor (108) für Gefäße (44) in dem Transportrohr (50) über den Isoliervorrichtungen (52) besitzt.

15. Vorrichtung nach einem beliebigen der Ansprüche 13 und 14, dadurch gekennzeichnet, daß die pneumatischen Transportvorrichtungen für die Gefäße (44) des weiteren eine zweite Leitung zur Luftzufuhr (104) besitzen, die mit den Druckluftzuführvorrichtungen über dritte, ein Ventil (106) bildende Vorrichtungen in Verbindung steht, die unmittelbar über den Isoliervorrichtungen (52) in das Transportrohr (50) einmünden.

## Claims

1. Installation for taking fluid samples in a confined area (10) located below a protective slab (12), said installation comprising a sampling vessel (14) located below the slab (12), at least one sampling needle (40) traversing a bottom of the slab and remote handling means for transferring individually the said sampling pots (44) into and out of the vessel (14) through the slab (2) and for engaging the pots (44) individually on the said needle (40) in order to perform a sampling operation, characterized in that the said remote handling means comprise:
a pot (44) transfer duct (60), traversing a slab (12) element (62), which is vertically mobile between an upper waiting position and a lower sampling position;
a drum (72) mounted in rotary manner beneath said slab (12) element (62), about an axis displaced with respect to that of the duct (60), and having a pot (44) reception nacelle (76) which can be brought, by the rotation of the drum, into an angular pot (44) reception and evacuation position, in which the nacelle is placed below the duct (60), and into at least one angular sampling position, in which the nacelle (76) can be brought above the sampling needle (40); and
means (102, 106) for the pneumatic transfer of the pots (44) upwards and downwards in the said duct (60).

2. Installation according to claim 1, characterized in that the remote handling means comprise several sampling needles (40) arranged along at least one circular arc, whose axis is displaced with respect to the axis of the drum (76) and a rotary plug (20) whose axis coincides with that of the circular arc and forming above the vessel (14) part of the slab (12) and in which is housed the slab element (62), so that in said angular sampling position of the nacelle (76), the latter can be brought above each of the needles by a rotation of the rotary plug.

3. Installation according to claim 1 or 2; characterized in that the remote handling means comprise rotation control means (82) for the drum (72) and installed on said slab element (62) so as to act on a control shaft (80) traversing the slab element in accordance with its axis and integral with the drum (72) at its lower end.

4. Installation according to claim 3, characterized in that the pneumatic transfer means for the pots (44) comprise an air supply pipe (98) which travels in said control shaft (80) and issues below the nacelle (76).

5. Installation according to claim 4, characterized in that the air supply pipe (98) is linked with ventilation means for the vessel (14) through first means forming a valve (100) and with compressed air supply means through second means forming a valve (102).

6. Installation according to any one of the preceding claims, characterized in that each sampling needle (40) is fitted into a receptacle (38) provided in the bottom of the vessel (14), so that the remote handling means comprise an anti-pull out member (88) fitted on the nacelle (76) so as to be displaceable thereon between an upper position in which said member (88) is flush with the lower end of the nacelle (76) and a lower position in which said member (88) is spaced from the lower end of the nacelle (76) by a distance substantially equal to the length of a part of each needle (40) projecting above the receptacle.

7. Installation according to claim 6, characterized in that the anti-pull out member (88) is moved towards its lower position by elastic means (96).

8. Installation according to claim 6 combined with either of the claims 4 and 5, characterized in that the air supply pipe (98) incorporates a rigid tube (92), slidingly housed within the control shaft (80) and which carries the anti-pull out member (88) at its lower end.

9. Installation according to any one of the claims 6 to 8, characterized in that the said slab element (62) has two cavities (120) whereof at least one can be brought above a needle (40) and under each of which can be brought the nacelle (76) as a result of the rotation of the drum (72), a gripping tool (118) being slidingly housed in each of the cavities (120) and is able to grasp a pot plug and a needle.

10. Installation according to claim 9, characterized in that each of the gripping tools incorporates a clamp (126) mounted at the lower end of a body (124) sliding in the cavity (120), said clamp (126) having at least one pivoting jaw (130), whose pivoting is controlled by a rod (132) able to slide in the body (124).

11. Installation according to any one of the preceding claims, characterized in that the bottom of the vessel (14) has a confinement member (42) able to seal the needle (40) passage orifice formed in the lower end of the nacelle (76), when the slab element (62) is in the lower position and when the nacelle (76) is placed below the pot transfer duct (60).

12. Installation according to any one of the preceding claims, characterized in that the remote handling means comprise a detector (114) of the presence of a pot (44) in the nacelle (76).

13. Installation according to any one of the preceding claims, characterized in that the remote handling means also incorporate isolating or insulating means (52) placed above the slab (12) and connected to the pot (44) transfer duct (60) by a flexible tube (54), said isolating means (52) being able to occupy an isolating position, in which the flexible tube (54) is isolated from a transfer tube (50) placed above the isolating means (52) and a passage position in which the flexible tube (54) and the transfer tube (50) are interlinked.

14. Installation according to claim 13, characterized in that the remote handling means comprise a second detector (108) of the passage of a pot (44) in the transfer tube above the isolating means (52).

15. Installation according to either of the claims 13 and 14, characterized in that the pneumatic pot (44) transfer means also have a second air supply pipe (104), which is linked with the compressed air supply means through third means forming a valve (106) and which issues into the transfer tube (50) immediately above the isolating means (52).
